**Europäisches Patentamt**

**European Patent Office**

**Office européen des brevets**

(19)

(11) Publication number : **0 057 110**
**B2**

(12)

# NEW EUROPEAN PATENT SPECIFICATION

(45) Date of publication of the new patent specification :
03.01.90

(21) Application number : 82300427.0

(22) Date of filing : 27.01.82

(51) Int. Cl.⁵ : **B 01 L   3/00**, G 01 N 33/52

(54) Reaction vessel and method for combining liquids and reagents.

(30) Priority : 28.01.81 US 229038

(43) Date of publication of application :
04.08.82 Bulletin 82/31

(45) Publication of the grant of the patent :
17.04.85 Bulletin 85/16

(45) Mention of the opposition decision :
03.01.90 Bulletin 90/01

(84) Designated contracting states :
DE FR GB

(56) References cited :
EP--A-- 0 014 797
EP--A-- 0 023 156
FR--A-- 2 325 920
US--A-- 4 227 810
Research Disclosure, Vol. 200, Dec. 1980, Abstract-
Nr. 22 (Kenneth Mason Publications Ltd., Homewell,
Havant, Hants, UK)

(73) Proprietor : **EASTMAN KODAK COMPANY (a New
Jersey corporation)**
**343 State Street**
**Rochester New York 14650 (US)**

(72) Inventor : **Columbus, Richard Lewis**
**Kodak Park**
**Rochester New York (US)**

(74) Representative : **Baron, Paul Alexander Clifford et al**
**Kodak Limited Patent Department Headstone Drive**
**Harrow Middlesex HA1 4TY (GB)**

## Description

The present invention relates to a reaction vessel and a method for effecting the interaction of liquids and reagents. A preferred use of the present invention is in the measurement of the amount of analytes in liquids.

In European Patent Application No. 79302338.3 (Publication No. 0 014 797), a liquid transport device is disclosed which comprises a liquid receiving zone in which opposed surfaces are spaced to provide capillary flow of liquid within the zone. Once a liquid is introduced into the receiving zone, it continues to flow between the opposed surfaces until the supply of liquid is exhausted, or until an edge of the zone is encountered. When the flow has stopped, there is no means for resuming the flow, except by adding more liquid to the device. The capability for stopping and then resuming flow is necessary for certain assays, such as in an assay using sequential reagents disposed at different locations in the device. However, the addition of more liquid to cause resumption of flow is not a useful technique when a particular assay requires that the liquid volume or concentration of analyte remain constant.

US-A-4 227 810 discloses a cuvette having two chambers interconnected by a passageway of relatively small cross-section at least a portion of which is of capillary size to prevent accidental mixture of liquid reactants in the chambers. To obtain mixing of the liquids in the respective chambers, a partial vacuum and air pressure are alternately applied to one of the chambers. The main problem in using such a cuvette in the performance of chemical analyses is that, unlike liquid transport devices relying on capillary flow, a relatively large amount of fluid is required to perform the analyses. The large amount of fluid makes it difficult to move the fluid from one of the chambers into the other chamber, except by the use of a strong vacuum or high air pressure. Further, once mixing between the two chambers has started, flow can occur in both directions in the passageway, even though it may be desirable to retain all of the fluid into one of the chambers.

It is an object of the present invention to overcome the aforementioned problems of prior-art devices by providing an improved apparatus and method for controlling the interaction of liquids and reagents, particularly for controlling such interaction in the performance of chemical analyses.

The present invention relates to a reaction vessel comprising members which form a first zone and a second zone for receiving a liquid, inlet means for admitting liquid into said first zone, and a passageway connecting said zones and having a cross section of capillary size, said members including opposed liquid transport surfaces and sidewall surfaces which cooperate with said opposed liquid transport surfaces to define said zones and said passageway, said opposed liquid transport surfaces being spaced apart a distance that is effective to induce capillary flow of said liquid at least in a portion of said first zone contiguous to said passageway (60, 60c-h), and said passageway is provided with meniscus control means having sharply defined opposed edges.

Such a reaction vessel, known per se from Research Disclosure, Vol. 200, Dec. 1980, Abstract-Nr. 22 (Kenneth Mason Publications Ltd., Homewell, Havant, Hants, UK), is characterized according to the invention in that at least said first zone contains a reagent capable of interacting with a material of a liquid introduced therein, that the meniscus control means additionally comprises said opposed sidewall surfaces or said opposed liquid transport surfaces within the passageway which converge in the direction of fluid flow and that said meniscus control means is for controlling the meniscus forming the leading edge of the capillary flow so as to stop the liquid from proceeding by capillary attraction from said first zone (22-22h) into said second zone (24, 24c-g, 160) and to permit flow of liquid into said second zone (24, 24c-g, 160) only when sufficient externally-generated pressure is applied to the liquid in the first zone (22-22h).

In accordance with the present invention there is also provided a method of combining together in a reaction vessel as defined above a reagent and a liquid containing a material, said method comprising the steps of

introducing a quantity of said liquid into said first zone and

transporting said liquid through said first zone by means of capillary action,

characterized by retaining said liquid with the meniscus forming the leading edge of the capillary flow contiguous to said passageway while a reagent in said first zone interacts with said material,

applying to said liquid in the first zone an externally-generated pressure sufficient to push the meniscus of the liquid from the first zone into the second zone, and

transporting said liquid through said second zone by means of capillary action.

The present invention is particularly useful in performing certain chemical analyses. For example, immunoassays are possible using the disclosed reaction vessel wherein the analyte analog is included in a first zone, preferably to compete with the unlabelled analyte of the liquid for a bonding reaction with a reagent therein. As used herein, « analog » refers to a labelled antigen corresponding to antigen to be assayed, or a labelled antibody corresponding to a particular antibody to be assayed. Selection of either an antibody or an antigen as the reagent of the first zone serves to immobilize a portion of both the analog and either the patient antigen or antibody, respectively.

Another advantage of the present invention is that it permits the analysis of whole blood. The reagent in the first zone of the reaction vessel can be a red cell-agglutinating reagent immovably adhered to the surfaces defining the first zone, and the second zone can contain a test element including at least one detection reagent specific to the analysis of the analyte of choice. The analyte is detectable from the plasma that is obtained from the first zone, following the separation of the red cells.

European Patent Application No. 80302501.4 (Publication No. 0 023 156) describes a capillary transport device having means for delaying liquid flow between two regions ; the liquid flow rate is retarded, but not stopped, by the delaying means.

Embodiments of the present invention will now be described, by way of example, with reference to the accompanying drawings in which :

Fig. 1 is a plan view of a reaction vessel constructed in accordance with the present invention, showing alternative design configurations in dashed lines ;

Fig. 2 is a section view, taken generally along the line II-II of Fig. 1, showing liquid in the first zone of the vessel ;

Fig. 3 is a section view, similar to that of Fig. 2, showing the liquid in the second zone of the vessel ;

Fig. 4 is a fragmentary plan view of a device similar to that of Fig. 1, but which is a comparative example rather than an embodiment of the present invention ;

Figs. 5, 6 and 7 are fragmentary section views similar to the view shown in Fig. 3, but illustrating alternative embodiments of the present invention ;

Figs. 8 and 9 are section views similar to that of Fig. 3, illustrating still further embodiments of the present invention ;

Fig. 10 is a plan view, similar to that of Fig. 1, illustrating a further embodiment of the present invention particularly adapted for the detection or measurement of antibodies or antigens ;

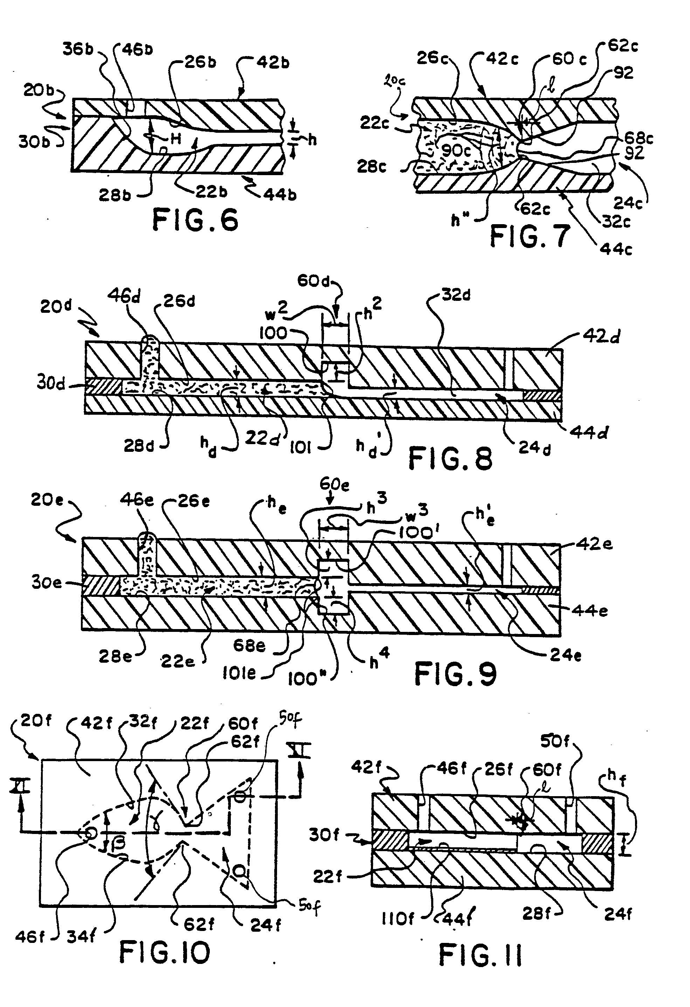

Fig.. 11 is a section view taken generally along the line XI-XI of Fig. 10 ;

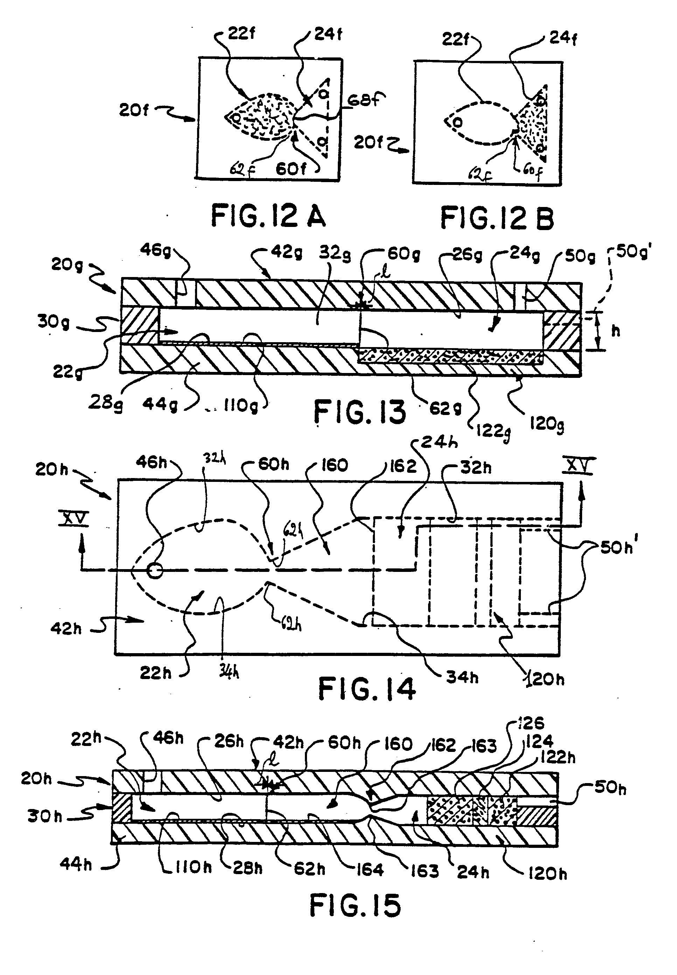

Figs. 12A and 12B are plan views of the device of Fig. 10, showing two stages in the use of the device ;

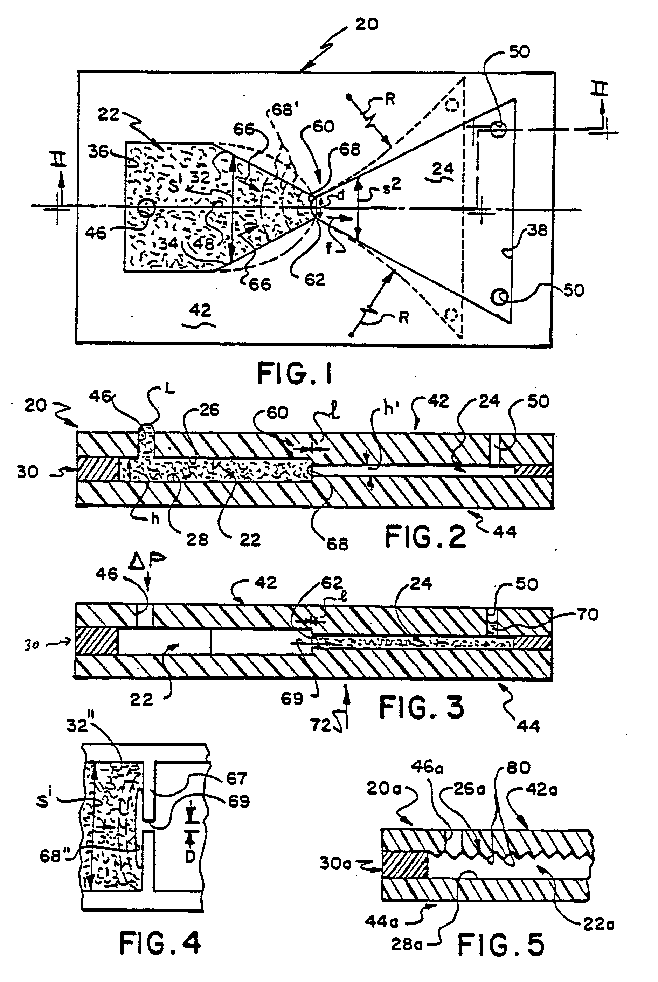

Fig. 13 is a section view, similar to that of Fig. 2, illustrating an embodiment of the present invention particularly adapted for detection of an analyte of whole blood ;

Fig. 14 is a plan view, similar to that of Fig. 12A, illustrating a three-zone embodiment of the present invention ; and

Fig. 15 is a section view taken generally along the line XV-XV of Fig. 14.

The present invention is described hereinafter with respect to the measurement or detection of analytes of biological liquids such as serum, plasma or whole blood. Liquids such as industrial liquids can also be analyzed using the vessel of the present invention. In addition, the vessel can be used to interact a liquid with a reagent for many other purposes.

In Figs. 1 and 2, there is shown a reaction vessel 20 comprising a first zone 22 and a second zone 24 adapted to receive liquid flow from first zone 22. Zones 22 and 24 are connected by means of a passageway 60 having a meniscus control means therein which will be explained in more detail hereinafter. Vessel 20 comprises a support member 44 and a cover member 42 superposed over member 44 and separated therefrom by a spacer member 30 (Fig. 2). Members 42, 44, and 30 are assembled together to form zones 22 and 24 ; members 42 and 44 are secured to spacer member 30 by any conventional means, for example a water-insoluble adhesive.

Within zones 22 and 24, members 42 and 44 provide, respectively, two opposed liquid transport surfaces 26 and 28, and spacer member 30 provides sidewall surfaces 32, 34, and end wall surfaces 36, 38 (Fig. 1). The location of sidewall surfaces 32, 34, and end wall surfaces 36, 38, are shown, for clarity, as solid lines in Fig. 1, such as they would appear if cover member 42 was transparent. Surfaces 26 and 28 (Fig. 2) are spaced apart a distance h in zone 22, and a distance h' in zone 24, both distances being selected to provide capillary flow of liquid. To ensure capillary flow of liquids, such as whole blood or blood serum, distance h should not exceed about 1 mm. To complete the transfer of all the liquid into second zone 24 once flow through passageway 60 has started and to retain liquid in zone 24, distance h' is preferably less than distance h. Specifically, a reduction in h' relative to h of at least 25 % is useful to induce substantially complete liquid transfer to zone 24 within a desirable length of time for most analyses. Most preferably, h is 125 μm, or less, to minimize the amount of liquid required for a reaction within the vessel, and h' is 94 μm or less. The selection of distance h' to be significantly less than h requires that the areas of surfaces 26 and 28 of second zone 24 be enlarged, relative to the areas of surfaces 26 and 28 in first zone 22, to ensure that all of the liquid of zone 22 will be accommodated in zone 24.

Additional zones (not shown) not having capillary spacing and therefore not adapted to receive liquid flow can be connected to second zone 24, for example adjacent to end wall surface 38.

To permit introduction of liquid into first zone 22, inlet means in the form of an aperture 46 is provided in cover member 42. A predetermined volume cf liquid is deposited at aperture 46, preferably in drop form. The size of aperture 46 is selected to ensure that the volume of liquid introduced will contact both surfaces 26 and 28 to initiate capillary transport of liquid through first zone 22. If 10 μl of liquid is the amount to be introduced for purposes of an intended test, aperture 46 is preferably about 1.0 mm to about 5.0 mm in diameter. Alternatively, aperture 46 is shaped to have cornered sidewalls (not shown), for example such that the shape in cross-section is hexagonal, to ensure more positive movement of the liquid into the aperture 46. To ensure the proper shape of the liquid wave front, or meniscus, and to prevent air entrapment, aperture 46 is preferably located at a point closer to end wall surface 36 than to sidewall surfaces 32 or 34, Fig. 1. Most preferably, it is located on the centre line, designated 48, between sidewall surfaces 32 and 34.

The reagent (not shown) with which the liquid is to interact in zone 22 is preferably coated, or otherwise bonded, such as by a chemical bond, onto any one of the exposed surfaces of zone 22, e. g., surfaces 26, 28, 32, 34 or 36. The reagent can be soluble or insoluble in the liquid to be reacted.

To permit air to be expelled from zones 22 and 24 ahead of an advancing liquid, apertures 50 are provided, preferably through cover member 42 in the vicinity of zone 24. Most preferably, one aperture 50 is located adjacent to the intersection formed by sidewall surface 32 with end wall surface 38, and a second aperture 50 is located adjacent the intersection formed by sidewall surface 34 with end wall surface 38. Such locations of apertures 50 permit full capillary flow in zone 24 with a minimum amount of resistance.

In accordance with an important aspect of the invention, a meniscus control means is provided in passageway 60 to control the liquid by stopping it from flowing from zone 22 to zone 24. As shown in Figs. 1 and 2, the meniscus control means in passageway 60 includes sidewall surfaces 32, 34, which converge in the direction of fluid flow, and sharply defined opposed edges 62, located between the zones 22 and 24 and spaced apart a distance « d ». The convergence of sidewall surfaces 32 and 34, provided by decreased spacing $s^1$, occurs preferably over at least the last 35 % of the flow path of liquid within zone 22, measured from aperture 46 to edges 62.

It is not certain what interaction between the liquid meniscus, designated 68 in Fig. 1, and the sidewall surfaces 32, 34, creates the phenomenon of meniscus stoppage. Tests have shown that the use of gradually, rather than abruptly, converging sidewall surfaces is preferred, together with edges 62 being limited to a radius of curvature no greater than about 0.02 cm. In this construction, edges 62 act as an energy barrier to capillary flow of liquid and specifically to advancement of meniscus 68. Flow of gas is not blocked, however, since passageway 60 is free of material that would block gas flow.

The convergence of sidewall surfaces 32 and 34 in zone 22 need not be at a constant rate. Alternatively, sidewall surfaces 32 and 34 can converge at an increasing rate as flow proceeds toward edges 62. In one embodiment of this alternative (shown as dashed lines for zone 22 in Fig. 1), the sidewall surfaces 32, 34, are concave and provide an increased volume for zone 22.

It is desirable that liquid flow stop at edges 62 when most, and preferably substantially all of the liquid, is within zone 22. That is, the volume of the liquid is selected to be consistent with the volume of zone 22. Only a negligible amount, if any (shown as portion L in Fig. 2) should project out of the reaction vessel. In, for example, immunoassay uses of the vessel, it is desirable that all of the predictable volume of liquid react in zone 22 with an immuno-reagent. For this reason, at least for tests involving constant liquid volumes, one of the members 42, 44, is preferably substantially rigid in both zones 22 and 24. As used herein, a « substantially rigid member » is a member having, when freely spanning a distance equal to the width or length of either zone 22, 24 of the vessel 20, and with atmospheric pressure on both sides of the member, a free-span deflection, hereinafter « sag », that does not exceed about 0.025 mm. In zone 22, if member 42 has any appreciable sag, one or both of the following could occur : The sagging portion could act as a « meniscus control means », prematurely stopping the meniscus before passageway 60 is reached. The sagging portion could also cause air entrapment in the vicinity thereof. In either case, the entire volume of added liquid would not flow into zone 22, and the interaction with the reagent of that zone would not occur to the desired extent.

Cover member 42 is preferably rigid in the area of zone 24 because sagging portions could again cause air entrapment. Such air entrapment could prevent transfer of all of the liquid from the first zone 22 to the second zone 24.

Although it is not critical to an understanding of the invention, it is believed that the shape of the meniscus 68 as it approaches the edges 62 is at least partially responsible for the stoppage of the meniscus 68. The shape of meniscus 68 at several positions in passageway 60 is shown by dashed lines 68' in Fig. 1. If meniscus 68 is angled to the sidewall surfaces 32, 34, of passageway 60, as shown by lines 68', it does stop at edges 62, in the absence of externally-generated pressures.

In contrast to the vessel 20 shown in Fig. 1, a comparative example is shown in Fig. 4 in which a wall 67 extends generally perpendicular to a sidewall surface 32" ; such an arrangement will not stop the capillary liquid flow. In the comparative example, an aperture 69 has a spacing D comparable to distance « d » in passageway 60 of vessel 20. Instead of being stopped, flow of liquid continues through aperture 69 at a retarded rate, as is described in the aforementioned European Patent Application No. 80302501.4 (Publication No. 0 023 156). In the example shown in Fig. 4, the meniscus 68" tends to have a shape, in the vicinity of aperture 69, that is roughly parallel to wall 67.

With reference to Fig. 1, sidewall surfaces 32 and 34 preferably extend from edges 62 into zone 24 with a configuration that aids in the emptying of all the liquid into zone 24 once flow starts past edges 62. (See Fig. 3.) The sidewall surfaces 32, 34 preferably diverge from edges 62 into zone 24 with a constantly increasing spacing $s^2$ (Fig. 1) or at an increasing rate (shown in dashed lines as convexly curved walls having a radius R).

The pressure ΔP (Fig. 3) needed to push the liquid meniscus 68 past edges 62 is an inverse function of the distance d. Accordingly, distance d is selected to provide sufficient stoppage of the meniscus 68 without requiring the use of excessive external pressures. A preferred value of d is about 0.04 cm. If distance h′ is about 0.03 cm, the distance from aperture 46 to edges 62 is about 0.9 cm, and d is about 0.04 cm, the pulse of pressure necessary to push a serum meniscus 68 past the edges 62 is about 80 Pa. Lesser values of d will require greater pressures to overcome the stoppage of flow at edges 62.

« Externally-generated pressure », as used herein, refers to any external pressure applied to the liquid, including a pressure applied to the liquid through the aperture 46, a vacuum applied to the liquid, or a pressure on the liquid resulting from a centrifugal force applied to the reaction vessel 20.

A further important aspect of this invention is the increased capillary attraction provided by the reduced distance h′ of second zone 24 (Fig. 2) ; it is this increased capillary attraction that causes completion of the transfer of liquid from zone 22 to zone 24, rather than the displaced air created by the externally-generated pressure. The advantage is that the capillary attraction is effective to provide complete transfer of liquid to second zone 24, even if the volume of liquid in first zone 22 deviates slightly from the expected volume. The use of pressure displacement to displace a given volume of liquid equal to the displaced air volume would not be readily adjustable for such liquid volume deviations. Therefore, the ΔP pressure applied to push the liquid meniscus 68 into zone 24 is only an impulse, the duration of which is by itself insufficient to transfer all the liquid. A duration of between about 1 and about 10 ms is sufficient in many cases to push the meniscus 68 into zone 24.

To minimize shear stresses as liquid is forced to flow past edges 62, the length 1 of edges 62 (Fig. 2) is minimized. Specifically, by providing sharp edges 62 with a negligible length in the direction of fluid flow, preferably no greater than 0.03 cm, the shear stresses that occur when liquid flows past edges 62 are minimized. The relatively low shear stresses permit the processing of whole blood, as is described hereinafter.

The preferred gas used to form the pulse of pressure ΔP is air. It will be appreciated that other gases are also useful, most preferably those that are inert relative to the liquid and the reagents used.

Although an immiscible liquid is useful also as a medium for delivering the pulse of pressure, gas is the preferred medium inasmuch as an immiscible liquid could move ahead of the reaction liquid and prevent complete transfer of the desired liquid to the second zone 24.

The behaviour of liquid introduced into zone 22 at aperture 46 will be apparent from the above description. That is, distance h is such that liquid introduced into zone 22 will flow within and through the zone 22, between the opposed liquid transport surfaces 26 and 28, by means of capillary attraction. A characteristic of passageway 60 is that the advancing liquid meniscus 68 stops at edge 62 even in those instances in which minor amounts L of liquid remain above aperture 46 (Fig. 2). The liquid remains temporarily stopped at edges 62 while any gas generated in zone 22 is free to flow into zone 24. Thereafter, if an externally-generated pressure pulse ΔP of air (Fig. 3) is applied to the liquid in zone 22, the energy barrier of edges 62 is overcome, and the liquid of zone 22 flows, in the direction of arrows 66, into zone 24. As noted above, the applied pressure is preferably insufficient to cause, absent other forces, a completion of the transfer of liquid from zone 22 to zone 24.

In accord with well-known principles of capillarity, the smaller spacing (distance h′) ensures that the liquid will preferentially flow into zone 24, emptying zone 22, and further, that the liquid will not return to zone 22. Air is pushed out through apertures 50 ahead of the advancing meniscus 68. The meniscus 68 stops when it reaches apertures 50, as shown at 70 (Fig. 3). Additional processing can then take place in zone 24 ; for example, a reaction with additional reagents, or a measurement of analyte in the liquid by an appropriate scan (arrow 72) using electromagnetic radiation that passes through either support member 44, as shown, or cover member 42 if exposed from above (not shown). If the direction of scan is as indicated by arrow 72, member 44 is preferably transparent, at least in the area of zone 24. If zone 22 is scanned also, the entire member 44 is preferably transparent.

Measurements conducted in the second zone 24 are selected to be compatible with the reagent and the interaction provided in the first zone 22. Useful examples of measurements include radiometric detection of a change in the liquid. Specifically, a photometer (not shown) or a fluorimeter (not shown) can be used to detect, respectively, the production of a dye density or a fluorescence. Also, measurements using the detection of radioactive labels are possible with vessel 20.

Specific useful materials for members 42, 44, and 30, include materials such as cellulose triacetate and materials, such as propypropylene, polystyrene, styrene-butadiene and ABS plastic, that are coated with a surfactant. These materials are sufficiently rigid at the desired thicknesses, for example, from about 0.018 to about 0.04 cm, for cover member 42. In addition, a flexible material such as a 25 μm polyethylene sheet is useful to provide the liquid transport surface 26 of the cover member 42 ; if used, the polyethylene sheet can be laminated to a sheet of one of the rigid materials noted above.

As an alternative to the use of a reduced value of h′, liquid can be preferentially and permanently emptied from zone 22 into zone 24 by the use of a surfactant, soluble in the liquid, that is coated on either or both of surfaces 26 and 28. The surfaces 26, 28, in this instance are formed from relatively non-wettable materials such as polystyrene. As used herein, « non-wettable » and « wettable » represent a condition best measured by the contact angle between the liquid and the surface in question. More specifically, a surface is generally considered non-wettable if the contact angle is greater than or equal to 90 degrees and wettable if it is less than 90 degrees. The coating (not shown) is applied to both zones 22 and 24. Useful surfactants include, for example, a nonylphenoxy polyglycidol surfactant obtainable from Olin Corp., Stamford, Conn., under the trade name « Olin 10G. » As long as the surfactant remains on the surfaces 26, 28, the liquid will wet the surfaces 26, 28, and zone 22 can be filled. However, the surfactant immediately dissolves from the surfaces 26, 28, or zone 22 when wetted. Therefore, after the liquid flows into zone 24, a thin film residue of liquid apparently forms and evaporates, and surfaces 26 and 28 of zone 22 revert back to being essentially non-wettable. Thus, the liquid cannot return to zone 22. In such an embodiment, any distance h' effective to ensure capillary flow into zone 24, such as 1 mm or less, is useful regardless of its relationship to distance h of zone 22. That is, the trailing edge (not shown) of liquid exiting zone 22 « sees » a surface condition that is essentially non-wettable. In contrast, the advancing meniscus 68 readily wets the surfactant-coated surfaces 26, 28. The result is an imbalance in driving forces, causing the liquid to empty from zone 22 into zone 24. Furthermore, the hydrophobic nature of the surfaces 26, 28, of now-emptied zone 22 is a sufficient energy barrier to return flow.

The previously-discussed embodiments are based on the assumption that, once the liquid has moved into the second zone 24, no significant amount of the liquid should remain in or flow back to the first zone 22, such as by a return flow past edges 62. However, in some instances it is desirable for liquid to remain in or return to the first zone 22 ; for example if the first zone 22 of the reaction vessel 20 is to be used to carry out an additional reaction on reaction products produced in the second zone 24. For this embodiment (not shown), distances h and h' between the opposed transport surfaces of the two zones 22, 24, are preferably equal, and preferably no water-soluble surfactant is used. An impulse of pressure $\Delta P$, if of short duration, as for the embodiments discussed above, will only move the liquid part way into the second zone 24 where it can react with a reagent in that zone. An additional reagent is immobilized in the first zone 22, the reagent being selected to react only with the reaction products produced by the reagent of the second zone 24. As each reaction takes place in its respective zone, a concentration gradient is created that induces diffusion of the necessary ingredient or reaction product to the zone where that reaction takes place.

In Fig 5, there is shown an alternative embodiment of the present invention in which parts similar to those previously described bear the same reference numeral to which the distinguishing suffix « a » has been attached. Thus, a vessel 20a comprises a cover member 42a, support member 44a, and spacer member 30a defining a first zone 22a, a second zone (not shown), and a meniscus control means as described above. Transport surface 26a, however, is provided with sawtooth ridges 80 spaced apart a regular distance. Preferably the ridges 80 are mutually parallel and extend perpendicularly to a bisector line (not shown) between the sidewalls (not shown). If surface 28a also has ridges (not shown), the ridges preferably extend at a positive angle to the ridges 80 of surface 26a in the manner taught by European Patent Application No. 79302338.3 (Publication No. 0 014 797).

An alternative embodiment of the present invention is shown in Fig. 6, in which parts similar to those previously described bear the same reference numeral to which the distinguishing suffix « b » is applied. In Fig. 6, a vessel 20b comprises a cover member 42b and a support member 44b, member 42b having an inlet aperture 46b for receiving liquid into a zone 22b. However, unlike previous embodiments, spacing H adjacent the aperture 46b is non-capillary, surfaces 26b and 28b being shaped to gradually converge to be spaced a capillary spacing h in the portion of zone 22b contiguous with the passageway (not shown). No sharp edges are present adjacent aperture 46b to create a barrier to flow. In this embodiment, liquid must be delivered to zone 22b with an initial pressure sufficient to move the liquid into that portion of zone 22b wherein the spacing between members 42b and 44b is a distance h ; capillary action then takes over to maintain flow towards the passageway (not shown). The initial pressure on the liquid is selected such that the pressure behind the liquid when it reaches the passageway (not shown), is significantly less than the $\Delta P$ pressure necessary to overcome the energy barrier of that passageway. End wall surface 36b is preferably sloped in this embodiment, to deflect incoming liquid to ensure that the liquid continues to advance to the point where capillary flow starts. The embodiment shown in Fig. 6 also demonstrates that the spacing between members 42b, 44b, here shoulder 30b, can be an integral part of member 44b, as shown, or of member 42b.

In Fig. 7, there is shown an alternative embodiment of the present invention in which parts similar to those previously described bear the same reference numeral to which the distinguishing suffix « c » is applied. Thus, vessel 20c comprises a cover member 42c and a support member 44c that respectively provide transport surface 26c and transport surface 28c ; members 42c and 44c are preferably formed from hydrophobic materials coated with a water-soluble surfactant as described above. A first zone 22c and a second zone 24c are separated by a passageway 60c which is provided with a meniscus control means, as described in a previous embodiment. However, in the embodiment shown in Fig. 7, the meniscus control means includes sharply defined edges 62c formed in the transport surfaces 26c and 28c that extend between a sidewall surface 32c and an opposite sidewall surface (not shown). The shape of transport surfaces 26c, 28c, preferably is such that the advancing meniscus 68c, as it moves through passageway 60c, extends generally perpendicularly (dashed lines 90) to the surfaces 26c, 28c; this shape of meniscus 68c apparently ensures that the edges 62c act as an energy barrier to further liquid flow. Most preferably, surfaces 26c, 28c, are shaped so that distance « h″ » between them is reduced at an increasing rate, in the vicinity of passageway 60c, to create opposed concavities immediately adjacent the edges 62c. As in previous embodiments, the length l of edges 62c in the direction of flow preferably does not exceed 0.03 cm. In this embodiment, the spacing between sidewall surface 32c and the opposite sidewall surface (not shown) is of no consequence to the stoppage of the meniscus 68c; such spacing can be selected to be constant or variable, as desired.

Preferably, zone 24c includes opposed surfaces 92 (here portions of surfaces 26c and 28c) that extend into zone 24c from passageway 60c at a separation distance that increases at a constant or at an increasing rate. Such a rate of separation helps to ensure that once the liquid meniscus 68c moves into zone 24c, all the liquid will flow into zone 24c.

Alternative embodiments are shown in Figs. 8 and 9 in which parts similar to those previously described bear the same reference numeral to which the distinguishing suffices « d » and « e », respectively, are appended. Thus, a vessel 20d and a vessel 20e are constructed in generally the same manner as the vessel 20 of Fig. 1, and include opposed liquid transport surfaces 26d, 28d, and 26e, 28e, respectively; first and second zones 22d, 24d, and 22e, 24e, respectively, are separated by meniscus control means. However, in the embodiment of Fig. 8, the meniscus control means comprises a channel 100 in transport surface 26d having a depth $h^2$ and a width $w^2$ that are sufficient to create an energy barrier to liquid flow, and thus, to stop flow. Specifically, the energy barrier comprises the sharp edge 101 created in surface 26d by the sudden depth $h^2$ of channel 100. In order to be effective, the channel 100 extends the full width between the zones. Most preferably, the depth $h^2$ of channel 100 is at least 0.02 mm greater than the spacing $h_d$ between surfaces 26d and 28d in zone 22d, and the width $w^2$ of channel 100 is greater than or equal to $h_d$. As in the embodiment of Fig. 1, distance $h_d'$ of zone 24d is significantly less than distance $h_d$ of zone 22d, and the total volumes of the two zones are adjusted to be generally equal. The radius of curvature of edge 101 preferably does not exceed about 0.02 cm.

In the embodiment of Fig. 9, the meniscus control means comprises a pair of opposed channels 100′ and 100″, with a depth $h^3$ and $h^4$, respectively, and a width $w^3$. To create the energy barrier to capillary flow, both edges 101e are sharp as before and the combined depth $(h^3 + h^4)$ is preferably at least 0.015 mm greater

than the distance $h_e$. Width $w^3$ need not have the same relationship to $h_e$ as $w^2$ has to $h_d$ in the embodiment of Fig. 8. Preferably $w^3$ is at least 25 μm. Distance $h_e'$ is at least 25 % less than $h_e$. A total depth $(h_e + h^3 + h^4)$ that is greater than a capillary spacing is also useful. The externally-generated pressure is selected to be large enough to move meniscus 68e into contact with zone 24e. A pressure of at least 80 Pa is useful for channels 100′ and 100″ having depths such that $h^3 + h^4 + h_e$ total about 0.415 mm, and a common width $w^3$ equalling 25 μm.

The reaction vessels 20-20e described above are useful to provide a variety of interactions in the first zone 22-22e between a reagent and material of the liquid, followed by analysis in the second zone 24-24e. As used herein, a « reagent » means any substance that is interactive with a material, such as an analyte, of the liquid, or a decomposition or reaction product of that material. « Interaction » and « interactive » refer to chemical reactions, catalytic activity such as enzymatic reactions, immunological reactions, or any other form of chemical or physical interaction that can result in the production of a useful result in the second zone. Preferred are those interactions that permit analysis of the material of the liquid. Highly preferred are binding reactions in which a reagent immobilized in the first zone 22-22e binds to at least a portion of the material of the liquid, thus retaining that bound portion in the first zone 22-22e. This binding reaction occurs whether the materials to be bound are labelled or unlabelled. If a vessel is to be used as an immunoassay device, as described hereinafter, both labelled analog and unlabelled analyte comprise the material that binds, in a competing fashion, to the reagent. If a vessel is to be used as a test device for whole blood, also as described hereafter, the materials that bind are the cells of whole blood, which need not be labelled for purposes of the test.

Preferably the reagent is immobilized on one or both of the sidewall surfaces, or the opposed transport surfaces, of the first zone 22-22e of the vessel 20-20e of the present invention. Alternatively, and particularly in those instances in which a relatively high volume of material is to interact with the immobilized reagent, the reagent is immobilized on filler such as beads as described in European Patent Application No. 79303004.0 (Publication No. 0 013 156). The beads (not shown) in turn are disposed in the first zone 22-22e and secured in place, for example by the use of a water-insoluble adhesive of the type described in the aforesaid European Patent Application No. 79303004.0 (Publication No. 0 013 156). In yet another embodiment (not shown) the beads are loosely disposed in the first zone, having a size that prevents them from passing through a passageway between the first zone and an adjacent zone; in this embodiment, distance « d » is substantially less than distance « h ».

As used herein, « immobilized » refers to a condition of immovable adherence created by any mechanism, such as chemical bonding, that is sufficient to withstand the pressures and stresses generated within a zone during use of a vessel or test device. A reagent is immobilized to one of the surfaces of a zone, or to inert fillers in the zone, if that reagent is bonded or adsorbed directly thereto, or if it is bonded via an intermediate, water-insoluble material that is itself secured to a zone.

Highly preferred reagents include those which produce immuno-reactions, such as antibodies or antigens, hereinafter « immunogens ». The material of the liquid in such a case is the corresponding immunogen, that is, the antigen or antibody, respectively. Any immuno-reaction is detectable, using the present invention, by the procedure hereinafter described. Immunological reagents, hereinafter « immunogen reagents », are preferably immobilized by being adsorbed or covalently bonded by any conventional procedure to any of the surfaces of a first zone, or to beads that are secured to a first zone.

One example of a useful procedure for covalently bonding an immunogen reagent is as follows : A polyacrylamide is selected to which the immunogen reagent is to be secured. This is either the material of a surface of a zone, or polyacrylamide beads adhered to a surface by a water-insoluble adhesive. The polyacrylamide in turn is partially hydrolyzed or hydrazinolyzed to permit covalent bonding by the antibodies. Details are described, for example, in US-A-3 853 987.

Indirect covalent bonding is also useful wherein a coupling agent is used having groups that will covalently bond with both the immunogen reagent and a surface of a zone (or beads secured to the zone). For example, covalent bonding of an immunogen reagent to ceramic beads is possible using the intermediate coupling agents described in US-A-3 652 761.

The selection of an immunogen as the reagent permits immunoassays to be conducted using the present invention. In Figs. 10-12B, there is disclosed an alternative embodiment which is adapted to such a use. Parts similar to those previously described bear the same reference numeral to which the distinguishing suffix « f » is applied. Thus, a vessel 20f is a test device comprising first and second zones 22f and 24f, formed by cover member 42f, support member 44f and spacer member 30f (Fig. 11). The two zones 22f, 24f, are connected by passageway 60f. As shown, passageway 60f is provided with a meniscus control means having two opposed edges 62f formed by sidewall surfaces 32f and 34f. Aperture 46f in member 42f permits liquid introduction and apertures 50f permit air venting. To minimize air entrapment, sidewall surfaces 32f and 34f are joined at an angle beta (β), and aperture 46f is on the bisector of angle β (Fig. 10). Angle β is preferably between about 7 and 75 degrees, and most preferably, 60 degrees. Sidewall surfaces 32f and 34f are curved toward each other in the area of passageway 60f to prevent the advancing wavefront from encountering edge discontinuities before encountering edges 63f. As edges 62f are neared, surfaces 32f and 34f converge at an increasing rate, consistent with the absence of edge discontinuities other than edge 62f. If angle β is about 60 degrees, then the angle of convergence gamma (γ) (Fig. 10) is preferably about 120 degrees.

Most preferably, an immunogen reagent for a particular complementary immunogen is immobilized in the first zone 22f, in the form, for example, of a coating 110f bonded to surface 28f of zone 22f (Fig. 11). Preferably, surface 26f bears a water-soluble surfactant (not shown) as discussed for previous embodiments. A known amount of the analog having an appropriate label is added to zone 22f either as an additional pre-applied reagent or as a liquid added with, to, or after the unknown sample. It will be appreciated that care is taken not to admix the analog and the immunogen reagent before adding the immunogen of the liquid to be assayed. The label is any detectable species, for example, an enzyme, a fluorescent species, or a radioactive species, chemically or physically linked (such as by adsorption) to the antigen or antibody. For example, a fluorescent species such as fluorescein is covalently bonded to an antigen. In a preferred embodiment, a polymeric latex bead is « loaded » with a fluorescent rare earth chelate, preferably a europium or terbium chelate. The resultant rare earth chelate-loaded latex bead is employed as a fluorescent label to which the analog of choice is physically adsorbed or covalently bonded. These latex polymer beads preferably have an average diameter of from about 0.01 to about 0.2 μm and are « loaded » with up to about 7.5 weight percent of the rare earth chelate. Because of the large number of rare earth chelate molecules which can be loaded into a single latex bead, the resultant label is highly fluorescent and provides immuno-fluorescence exhibiting excellent sensitivity. A highly-preferred analog is one employing a fluorescent, rare earth chelate-loaded polymeric latex bead as described in European Patent Application No. 78300913.7 (Publication No. 0 002 963).

While the liquid is retained in the first zone as shown by the location of meniscus 68f (Fig. 12a) the analog and the immunogen to be assayed compete for binding to the immunogen reagent which is immobilized in the first zone 22f of the vessel. After an appropriate length of time, for example, 5 to 15 minutes, a pulse of pressure such as 80 Pa is applied to force the liquid to empty from the first zone 22f into the second zone 24f (Fig. 12b) carrying immunogen and analog that have not become bound to the immunogen reagent. Useful methods of measurement to determine the concentration of immunogen include : (A) detecting the unbound analog which has flowed into the second zone, and/or (B) detecting the analog which remains bound to the immobilized immunogen reagent in the first zone. In either method, the amount of immunogen in the liquid sample is determined based on the detected concentration of analog in accordance with conventional immunological techniques. Such techniques are set forth in European Patent Application No. 79303004.0 (Publication No. 0 013 156).

It has been determined that the ionic bond formed in an immuno-reaction is sufficient to withstand the shear stress generated by the pulse of pressure used to force plasma, for example, having a viscosity of 2.4 mPa.s, to flow from zone 22f into zone 24f through passageway 60f. That is, under less than the best conditions, the shear force likely to occur can be shown to be about $2 \times 10^{-14}$ N/molecule. The energy bond strength can be shown to be about $10^{-10}$ N/molecule, more than enough to resist the stress.

An avantage of vessel 20f is that the concentration determined by the measurement made in zone 24f of free analog, can be checked by measuring the bound analog in zone 22f.

The present invention is useful in performing analyses of whole blood. In such an application, the immobilized reagent is selected to be a red cell-agglutinating reagent. Such a reagent attracts or binds substantially all the red blood cells of whole blood added to the device, leaving only the plasma free to flow into a second zone of a reaction vessel, such as vessels 20-20f, when an externally-generated pulse of pressure is delivered to the liquid in the manner described above. The necessary reagents for analyzing the analyte of the plasma are contained in the second zone.

Useful examples of such reagents for immobilizing the red cells include agglutinating reagents such as positively-charged polymers, e. g., polybrene ; lectins ; high molecular weight dextrans ; and phytohemagglutinin as described in US-A-3 146 163, and US-A-3 902 964. The last-noted materials are proteinaceous, and as such are subject to bonding to appropriate surfaces, such as polyacrylamide, by the procedures described above. Thus, agglutinating agents are readily immobilized within a reaction vessel and/or to beads secured in the vessel.

In Fig. 13, there is disclosed an alternative embodiment useful as a test device for the measurement of an analyte of whole blood. Parts similar to those previously described bear the same reference numeral to which the distinguishing suffix « g » is applied. Thus, a vessel 20g comprises cover and support members 42g and 44g, and a spacer member 30g ; members 42g, 44g, and 30g are assembled together to form a first zone 22g and a second zone 24g with a passageway 60g between zones 22g and 24g. Liquid is introduced into zone 22g through aperture 46g, and apertures 50g are provided for the venting of air from zone 24g. Also, as in the device of Fig. 11, a reagent coating 110g is applied to at least one of the members forming zone 22g directly, or to beads (not shown) adhered within the zone. Surface 26g of both zones 22g and 24g preferably bears a water-soluble wetting agent or surfactant (not shown), and the material of members 42g, 44g, and 30g is selected to be relatively non-wetting. The beads, if used, preferably occupy less than 50 % of the volume of zone 22g. In this case, the reagent is an agglutinating agent such as phytohemagglutinin, immobilized as described above.

Alternatively, vessel 20g is useful if the vent apertures are formed in spacer member 30g, as shown by the dashed lines 50g' of Fig. 13. Preferably, such apertures 50g' are formed by notching the top of spacer member 30g.

To permit analysis of the plasma as it enters zone 24g in response to a pulse of pressure, an analyte detection element 120g is provided in or adjacent to member 44g in zone 24g. Preferably the detection element comprises one or more detection reagent layers 122g having a variety of binder compositions and a variety of detection reagents. For example, gelatin, cellulose acetate, polyvinyl alcohol, agarose and the like are useful binders, the degree of hydrophilicity of the layer being dependent upon the material selected.

Additional layers, disposed above layer 122g, are useful to provide a variety of chemistries or functions. If used, these additional layers provide additional detection reagents, or filtering, registration and/or mordanting functions, such as are described in US-A-4 042 335.

As used herein, « detection reagent » means a material that is capable of interaction with an analyte, a precursor of an analyte, a decomposition product of an analyte, or an intermediate of an analyte, to ultimately produce a detectable response. Useful detection elements in many instances include a first detection reagent that produces from the analyte an intermediate or a decomposition product, and a second detection reagent, labelled « indicator » because of its function, that is responsive to the intermediate or decomposition product to produce said change. Useful detection reagents also include preformed, radiometrically detectable species that are caused by the analyte of choice to move out of a radiometrically-opaque portion or layer of the detection element and into a radiometrically-transparent portion or layer, such as a registration layer which can be layer 122g in contact with member 44g.

The noted interaction between a detection reagent and the analyte therefore includes chemical reactions, catalytic activity as in the formation of an enzyme-substrate complex, or any other form of chemical or physical interaction, including physical displacement, that can produce ultimately a detectable response in the test element. The assay method is designed to produce a response signal that is predictably related to the amount of analyte that is present.

The preferred detection element is designed to measure radiometric responses, produced by impinging electromagnetic energy on the element. As is well known, radiometric detection includes both colorimetric and fluorimetric detection, the method used depending upon the detection reagents selected as the indicator.

The detection element 120g is adaptive to a variety of different analytes. Preferably, the assays are all oxygen-independent, as the flow of liquid into the zone 24g tends to seal off detection element 120g from any additional oxygen. Typical analytes which can be tested include total protein, bilirubin and the like. Useful detection reagents and binder or vehicle compositions for, e. g., layer 122g and any additional layers of the element include those described in US-A-4 132 528, and US-A-4 069 016 or US-A-4 069 017. Detection element 120g is also useful to test for other analytes.

The method of using vessel 20g will be apparent from the preceding discussion. A quantity of whole blood, for example, a drop, is added to zone 22g via aperture 46g, and the meniscus flows up to edges 62g of passageway 60g, where it stops. Coating 110g comprises a red cellagglutinating reagent which immobilizes the red cells while the liquid remains in zone 22g. After an appropriate length of time, such as 10 minutes, the plasma is separable from the cells by applying an impulse of pressure, such as a pressure of about 80 Pa, to aperture 46g. The plasma flows into zone 24g and into detection element 120g. After an incubation period of up to about five to ten minutes, a radiometric signal is projected onto element 120g which is detected by a suitably calibrated radiometer (not shown).

Another alternative embodiment of the present invention is shown in Figs. 14 and 15. Parts similar to those previously described bear the same reference numeral to which the distinguishing suffix « h » is added. Thus, a vessel 20h comprises cover and support members 42h and 44h fixed to a spacer member 30h. Aperture 46h in member 42h permits introduction of liquid to a first zone 22h having a coating 110h on a surface 28h thereof (Fig. 15). Coating 110h comprises a cell-agglutinating reagent bonded to surface 28h, as described for the embodiment shown in Fig. 13. Surface 26h is preferably coated with a water-soluble surfactant (not shown), and members 42h, 44h and 30h are relatively non-wetting. Passageway 60h is provided with a meniscus control means which includes opposed, sharply-defined sidewall edges 62h (Fig. 15) ; the meniscus control means serves to confine the whole blood within zone 22h to allow the red blood cells to become bound.

In addition to first zone 22h, vessel 20h comprises second zone 160 and a third analysis zone 24h disposed downstream from zone 22h. Each of the zones 22h, 160, 24h, is of approximately equal volume and is separated from the adjacent zone by meniscus control means which include edges 62h in passageway 60h and opposed edges 163 in passageway 162 (Fig. 15). Edges 163 are located on the transport surfaces 26h, 28h, to avoid the necessity of gradually increasing the spacing of sidewall surfaces 32h and 34h adjacent the detection element 120h (Fig. 14).

Zone 160 preferably includes a coating 164 on surface 28h (Fig. 15) of a detection reagent in addition to those present in detection element 120h located in zone 24h. As shown, detection element 120h is a multi-layered element the layers of which are stacked in the direction of the flow of liquid into zone 24h. In a preferred form, detection element 120h comprises at least layer 122h, and optionally, a detection reagent layer 126 and a barrier composition layer 124. The barrier composition is uniformly permeable to a gas such as $NH_3$, but is substantially impermeable, over the time of the test, to interferents such as are carried by water. Because of this arrangement, a vent aperture 50h is preferably located in spacer member 30h.

A preferred use of vessel 20h is as a test device for plasma ammonia alone, or for plasma ammonia followed by plasma creatinine. Coating 164, for these uses, comprises a buffer selected to maintain a pH of about 9.0 in the plasma to release dissolved ammonia. A preferred buffer is a mixture of tris buffer, comprising tris-(hydroxymethylaminomethane), and $KH_2PO_4$. Layer 122h of element 120h comprises an indicator responsive to ammonia that changes colour in proportion to the amount of $NH_3$ gas that flows into layer 122h from zone 160 while the plasma is being retained in zone 160. For example, bromophenol blue is useful as the indicator.

Alternatively, vessel 20h further includes a detection reagent, preferably in layer 126, that decomposes creatinine into $NH_3$ gas, and the described barrier composition, preferably in layer 124, that allows the $NH_3$ but not the liquid of the plasma to permeate. Useful compositions for layers 124 and 126, as well as layer 122h, are described in US-A-4 276 377.

The above-described alternative construction of vessel 20h and detection element 120h is used first to test for plasma ammonia, and then, optionally, to test for plasma creatinine. After the plasma is forced into zone 160, coating 164 serves to release plasma ammonia as $NH_3$ gas which flows into zone 24h while the plasma is stopped at edges 162. The $NH_3$ permeates indicator layer 122h of detection element 120h to produce a detectable colour change. After the colour change, which is proportional to the plasma ammonia, is detected, the plasma is analyzed for creatinine content. For the creatinine test, a second impulse of air pressure is applied at aperture 46h to push the liquid meniscus past edges 162, into zone 24h and then into detection element 120h. The additional $NH_3$ generated from the decomposition of creatinine is detectable by the same indicator used for the plasma ammonia. To use vessel 20h as a test device only for plasma ammonia, the second impulse of pressure is simply omitted.

Yet another alternative embodiment of the invention (not shown) comprises the device of Fig. 15 wherein zone 22h is omitted and a previously obtained plasma is placed via an inlet aperture, similar to aperture 46h, directly into zone 160. The procedure for the sequential detection of plasma ammonia and/or creatinine is as described above.

## Claims

1. A reaction vessel (20-20h) comprising members (42-42h, 44-44h, 30, 30a, b, d-h) which form a first zone (22-22h) and a second zone (24, 24c-g, 160) for receiving a liquid, inlet means (46, 46a, b, d-h) for admitting liquid into said first zone (22-22h), and a passageway (60, 60c-h) connecting said zones (22-22h, 24, 24c-g, 160) and having a cross section of capillary size, said members (42-42h, 44-44h, 30, 30a, b, d-h) including opposed liquid transport surfaces (26-26h, 28-28h) and sidewall surfaces (32, 32c, d, f-h, 34, 34f, h which cooperate with said opposed liquid transport surfaces (26-26h, 28-28h) to define said zones (22-22h, 24, 24c-g, 160) and said passageway (60, 60c-h), said opposed liquid transport surfaces (26-26h, 28-28h) being spaced apart a distance that is effective to induce capillary flow of said liquid at least in a portion of said first zone (22-22h) continuous to said passageway (60, 60c-h), and said passageway (60, 60c-h) is provided with meniscus control means (62, 62c, f-h, 100, 100′, 100″) having sharply defined opposed edges (62, 62f-h), characterized in that at least said first zone (22-22h) contains a reagent capable of interacting with material of a liquid introduced therein, that the meniscus control means additionally comprises said opposed sidewall surfaces (32, 32c, d, f-h, 34, 34f, h) or said opposed liquid transport surfaces (26c, 28c) within the passageway which converge in the direction of fluid flow, and that said meniscus control means is for controlling the meniscus forming the leading edge of the capillary flow so as to stop the liquid from proceeding by capillary attraction from said first zone (22-22h) into said second zone (24, 24c-g, 160) and to permit flow of liquid into said second zone (24, 24c-g, 160) only when sufficient externally-generated pressure is applied to the liquid in the first zone (22-22h).

2. A reaction vessel (20-20h) according to claim 1, characterized in that one of said members (42-42h) is substantially rigid.

3. A reaction vessel (20-20h) according to claim 1 or 3, characterized in that said distance is effective to induce capillary flow of said liquid from said inlet means (46, 46a, b, d-h) to said passageway (60, 60c-h).

4. A reaction vessel (20-20h) according to any one of claims 1-3, characterized in that at least one of said opposed surfaces (26-26h, 28-28h) comprises, in either or both of said zones (22-22h, 24, 24c-g, 160), a relatively non-wettable material coated with a surfactant soluble in the liquid and capable of making said surfaces wettable by the liquid.

5. A reaction vessel (20-20b, 20f-h) according to claim 1, characterised in that said sidewall surfaces (32, 32f-h, 34, 34f, h) in said second zone (24, 24f-g, 160) adjacent to said passageway (60, 60f-h) diverge from said edges (62, 62f-h) into said second zone (24, 24f-g, 160) at a constant rate.

6. A reaction vessel (20-20b, 20f-h) according to claim 1, characterized in that said sidewall surfaces (32, 32f, 34, 34f) of said second zone (24, 24f) diverge from said edges (62, 62f) at a rate which increases with distance from said edges (62, 62f).

7. A reaction vessel (20c) according to any one of claims 1-4, characterized in that said meniscus control means comprises a pair of opposed edges (62c) in said two opposed surfaces (26c, 28c) that provide a reduction in said spaced-apart distance (h″).

8. A reaction vessel (20c) according to claim 7, characterized in that said distance reduction between said two surfaces (26c, 28c) increases in rate as said meniscus control means (62c) is approached from said inlet means.

9. A reaction vessel (20, 20c) according to any one of claims 1-8, characterized in that said meniscus control means includes opposed edges (62, 62c) having a length in the direction of liquid flow therethrough that is no greater than 0.03 cm.

10. A reaction vessel (20, 20a, b, d, e) according to any one of claims 1-4, characterized in that said members (42, 42a, b, d, e, 44, 44a, b, d, e, 30, 30a, b) which form said zones (22, 22a, b, d, e, 24, 24d, e) include a support member (44, 44a, b, d, e) and a cover member (42, 42a, b, d, e) which are spaced apart a first distance ($h'$, $h_d'$, $h_e'$) in said second zone (24, 24d, e) and a second distance ($h$, $h_d$, $h_e$) in said first zone (22, 22a, b, d, e), said first distance ($h'$, $h_d'$, $h_e'$) being less than said second distance ($h$, $h_d$, $h_e$) by an amount effective to preferentially attract said liquid, in response to said application of pressure on the liquid, into said second zone (24, 24d, e) from said first zone (22, 22a, b, d, e) and to confine the liquid thus attracted within the second zone (24, 24d, e).

11. A reaction vessel (20d, e) according to any one of claims 1-4, characterized in that said meniscus control means comprises a channel (100, 100′, 100″) in at least one of said opposed surfaces (26d-e, 28d-e) having a depth ($h^2$, $h^3$, $h^4$) and width ($w^2$, $w^3$) sufficient to stop the meniscus from flowing into said second zone (24d, e).

12. A reaction vessel (20d) according to claim 11, characterized in that said channel (100) has a depth ($h^2$) which is at least 0.02 mm greater than the distance ($h_d$) between said two opposed surfaces (26d, 28d) in said first zone (22d).

13. A reaction vessel (20e) according to claim 11, characterized in that said meniscus control means comprises two opposed channels (100′, 100″) having a combined depth ($h^3 + h^4$) that is at least 0.015 mm greater than the distance ($h_e$) between said two opposed surfaces (26e, 28e) in said first zone (22e).

14. A reaction vessel (20f) according to claim 1, characterized in that said reagent is in an immobilized state in said first zone (22f) and has the capability of binding with a material of the liquid and with a labelled material, said second zone (24f) being adapted to receive liquid and material contained therein that is not bound to said reagent from said first zone (22f).

15. A reaction vessel (20f) according to claim 14, characterized in that said material is an immunogen present in a biological liquid, said reagent is the immunogen complement, and said second zone (24f) is adapted for detection of labelled material.

16. A reaction vessel (20f) according to claim 15, characterized in that both of said zones (22f, 24f) are adapted for detection of labelled material.

17. A reaction vessel (20g) according to claim 1, characterized in that said first zone (22g) contains a cell-agglutinating reagent immovably adhered to at least one of i) said opposed surfaces and ii) spacer members (30g), said inlet means (46g) permitting the introduction of whole blood into said first zone (22g) and said second zone (24g) is adapted to receive plasma from said first zone (22g) and contains a detection element (120g) including at least one detection reagent specific to the analysis of an analyte of the plasma.

18. A reaction vessel (20h) according to claim 1, characterized in that a third zone (24h) is disposed downstream from said second zone (160) and a second meniscus control means (163) is located in a passageway (162) disposed between said third zone (24h) and said second zone (160), and in that said first zone (22h) contains a cell-agglutinating reagent immovably adhered to at least one of i) said opposed surfaces and ii) spacer members (30h), said inlet means (46h) permitting the introduction of whole blood into said first zone (22h), said second zone (160) is adapted to receive plasma from said first zone (22h), and said third zone (24h) contains a detection element (120h) including at least one detection reagent specific to the analysis of an analyte of the plasma.

19. A reaction vessel (20h) according to claim 18, characterized in that said detection element (120h) comprises detection reagents sufficient for the generation of a colour change proportional to the amount of plasma ammonia as well as the amount of plasma creatinine that are present in the whole blood.

20. A method of combining together in a reaction vessel according to any one of claims 1-19 a reagent and a liquid containing a material, said method comprising the steps

of introducing a quantity of said liquid into said first zone and

transporting said liquid through said first zone by means of capillary action,

characterized by retaining said liquid with the meniscus forming the leading edge of the capillary flow contiguous to said passageway while a reagent in said first zone interacts with said material,

applying to said liquid in the first zone an externally-generated pressure sufficient to push the meniscus of the liquid from the first zone into the second zone, and

transporting said liquid through said second zone by means of capillary action.

21. A method according to claim 20, characterized in that said reagent is capable of bonding with said material and is immobilized in said first zone, said quantity of liquid introduced into said first zone contains i) a liquid containing an unknown amount of unlabelled material, and ii) a liquid containing a known amount of labelled material, said quantity of liquid is retained within said first zone while the reagent binds with said labelled material and said unlabelled material, said pressure is sufficient to force the liquid and the material therein that is free of said reagent to start flowing into said second zone, and further comprising the step of detecting the free labelled material in said second zone after the liquid has moved into the second zone.

22. A method according to claim 21, characterized in that instead of said last recited step, the bound immobilized labelled material in said first zone is detected.

23. A method according to claim 21, characterized in that said material is an immunogen found in a biological liquid, and said reagent is the complement of said immunogen.

24. A method according to claim 21, 22 or 23, followed by the steps of

comparing the amount of labelled material detected against said known amount, and

calculating the amount of unknown, unlabelled material that competed with the labelled material for said reagent.

25. A method according to claim 20, characterized in that said liquid is whole blood and said material is an analyte of whole blood, said reagent is a cell-agglutinating reagent that is immobilized in said first zone, said second zone contains at least one detection reagent specific to said analyte, said whole blood is retained in said first zone a length of time sufficient to cause blood cells to bind to said cell-agglutinating reagent and thus to separate from the blood plasma, said pressure is sufficient to force the plasma, but not the cells, to flow into said second zone, and further comprising the step of detecting within said second zone a radiometric change resulting from the interaction between said detection reagent and said analyte.

26. A method according to claim 25, characterized by the further step of forcing said plasma into a third zone of the vessel between said first and second zones before it is forced into said second zone, the third zone containing a gas-releasing reagent so that gas is released from the plasma before the plasma is forced into said second zone.

27. A method according to claim 20, characterized in that said reagent is an ammonia gas-releasing reagent and is in said first zone, said second zone includes i) a detection reagent that decomposes creatinine into at least ammonia gas and ii) an indicator that generates a radiometrically detectable change in response to said gas, said liquid is plasma containing ammonia and creatinine, said plasma is retained in said first zone while said ammonia gas is released by said gas-releasing reagent and allowed to flow into said second zone, said pressure is sufficient to force the plasma to start flowing into said second zone, and further comprising the steps of

detecting the gas in said second zone with said indicator before applying said pressure, and

as said plasma is being transported through said second zone, reacting (i) the plasma with said detection reagent to form additional ammonia gas, and (ii) the additional gas with said indicator, whereby the concentration of creatinine is detected.

28. A method according to claim 27, characterized by the further steps, performed prior to the previously-recited steps, of

placing a quantity of whole blood within a third zone of the vessel disposed adjacent to and in fluid communication with said first zone

retaining said quantity of whole blood in said third zone a length of time sufficient to cause blood cells to bind to a cell-agglutinating reagent that is immobilized in said third zone and thus to separate from the blood plasma ;

applying to the third zone an externally-generated pressure in an amount sufficient for introducing said plasma into said first zone.


## Patentansprüche

1. Reaktionsgefäß (20-20h) mit Elementen (42-42h, 44-44h, 30, 30a, b, d-h), die eine erste Zone (22-22h) und eine zweite Zone (24, 24c-g, 160) für die Aufnahme einer Flüssigkeit bilden, mit Einlaßmitteln (46, 46a, b, d-h) für die Einführung von Flüssigkeit in die erste Zone (22-22h) und einer die Zonen (22-22h, 24, 24c-g, 160) verbindenden Engstelle (60, 60c-h) mit einem Querschnitt kapillarer Größe, wobei die Elemente (42-42h, 44-44h, 30, 30a, b, d-h) einander gegenüberliegende Flüssigkeitstransportflächen (26-26h, 28-28h) und Seitenflächen (32, 32c, d, f-h, 34, 34f, h) aufweisen, die mit den einander gegenüberliegenden Flüssigkeitstransportflächen (26-26h, 28-28h) zusammen die Zonen (22-22h, 24, 24c-g, 160) und die Engstelle (60, 60c-h) bilden, wobei die einander gegenüberliegenden Flüssigkeitstransportflächen (26-26h, 28-28h) in einem Abstand voneinander angeordnet sind, der eine kapillare Strömung der Flüssigkeit in mindestens einem an die Engstelle (60, 60c-h) angrenzenden Teil der ersten Zone (22-22h) bewirkt, und die Engstelle (60, 60c-h) mit Meniskussteuermitteln (62, 62c, f-h, 100, 100′, 100″) versehen ist, die einander gegenüberliegende scharfe Kanten (62, 62f-h) besitzen, dadurch gekennzeichnet, daß mindestens die erste Zone (22-22h) ein Reagens enthält, das zu einer Interaktion mit einem Material einer in diese Zone eingeführten Flüssigkeit fähig ist, daß die Meniskussteuermittel innerhalb der Engstelle außerdem die einander gegenüberliegenden Seitenflächen (32, 32c, d, f-h, 34, 34f, h) oder die einander gegenüberliegenden Flüssigkeitstransportflächen (26c, 28c) umfassen, die in der Strömungsrichtung der Flüssigkeit konvergieren, und daß die Meniskussteuermittel den die Vorderkante der Kapillarströmung bildenden Meniskus so steuern, daß die Flüssigkeit in ihrer durch kapillare Anziehung bewirkten Bewegung von der ersten Zone (22-22h) in die zweite Zone (24, 24c-g, 160) angehalten wird und ein Einströmen von Flüssigkeit in die zweite Zone (24, 24c-g, 160) nur möglich ist, wenn auf die Flüssigkeit in der ersten Zone (22-22h) von außen ein ausreichend starker Druck ausgeübt wird.

2. Reaktionsgefäß (20-20h) nach Anspruch 1, dadurch gekennzeichnet, daß eines der Elemente (42-42h) im wesentlichen starr ist.

3. Reaktionsgefäß (20-20h) nach Anspruch 1 oder 2, dadurch gekennzeichnet, daß der Abstand eine Kapillarströmung der Flüssigkeit von den Einlaßmitteln (46, 46a, b, d-h) zu der Engstelle (60, 60c-h) bewirkt.

4. Reaktionsgefäß (20-20h) nach einem der Ansprüche 1-3, dadurch gekennzeichnet, daß mindestens eine der einander gegenüberliegenden Flüssigkeitstransportflächen (26-26h, 28-28h) in einer der Zonen oder in beiden Zonen (22-22h, 24, 24c-g, 160) ein relativ unbenetzbares Material aufweist, das mit einem oberflächenaktiven Mittel beschichtet ist, das in der Flüssigkeit lösbar ist und bewirkt, daß die Flüssigkeitstransportflächen mit der Flüssigkeit benetzbar sind.

5. Reaktionsgefäß (20-20b, 20f-h) nach Anspruch 1, dadurch gekennzeichnet, daß sich die Seitenflächen (32, 32f-h, 34, 34f, h) in der zweiten Zone (24, 24f-g, 160) benachbart der Engstelle (60, 60f-h) von den Kanten (62, 62f-h) aus in einem konstant zunehmenden Winkel divergierend in die zweite Zone (24, 24f-g, 160) hinein erstrecken.

6. Reaktionsgefäß (20-20b, 20f-h) nach Anspruch 1, dadurch gekennzeichnet, daß die Seitenflächen (32, 32f, 34, 34f) der zweiten Zone (24, 24f) von den Kanten (62, 62f) aus in einem Winkel divergieren, der mit der Entfernung von den Kanten (62, 62f) zunehmend größer wird.

7. Reaktionsgefäß (20c) nach einem der Ansprüche 1-4, dadurch gekennzeichnet, daß die Meniskussteuermittel zwei in den beiden einander gegenüberliegenden Flüssigkeitstransportflächen (26c, 28c) vorgesehene einander gegenüberliegende Kanten (62c) umfassen, die den Abstand (h″) zwischen diesen Flächen verringern.

8. Reaktionsgefäß (20c) nach Anspruch 7, dadurch gekennzeichnet, daß der Abstand zwischen den beiden Flüssigkeitstransportflächen (26c, 28c) von den Einlaßmitteln aus zu den Meniskussteuermitteln (62c) hin zunehmend geringer wird.

9. Reaktionsgefäß (20, 20 g) nach einem der Ansprüche 1-8, dadurch gekennzeichnet, daß die Meniskussteuermittel einander gegenüberliegende Kanten (62, 62c) besitzen, deren Länge in Richtung der durch sie hindurchgehenden Flüssigkeitsströmung höchstens 0.03 cm beträgt.

10. Reaktionsgefäß (20, 20a, b, d, e) nach einem der Ansprüche 1-4, dadurch gekennzeichnet, daß die die Zonen (22, 22a, b, d, e, 24, 24d, e) bildenden Elemente (42, 42a, b, d, e, 44, 44a, b, d, e, 30, 30a, b) ein Trägerelement (44, 44a, b, d, e) und ein Deckelement (42, 42a, b, d, e) enthalten, die in der zweiten Zone (24, 24d, e) in einem ersten Abstand ($h'$, $h_d'$, $h_e'$) und in der ersten Zone (22, 22a, b, d, e) in einem zweiten Abstand ($h$, $h_d$, $h_e$) voneinander angeordnet sind, wobei der erste Abstand ($h'$, $h_d'$, $h_e'$) um einen solchen Betrag kleiner ist als der zweite Abstand ($h$, $h_d$, $h_e$), daß dadurch vorzugsweise Flüssigkeit unter der Wirkung von Druck aus der ersten Zone (22, 22a, b, d, e) in die zweite Zone (24, 24d, e) strömt und die so eingeströmte Flüssigkeit innerhalb der zweiten Zone (24, 24d, e) gehalten wird.

11. Reaktionsgefäß (20d, e) nach einem der Ansprüche 1-4, dadurch gekennzeichnet, daß die Meniskussteuermittel in mindestens einer der einander gegenüberliegenden Flüssigkeitstransportflächen (26d-e, 28d-e) einen Kanal (100, 100', 100") aufweisen, dessen Tiefe ($h^2$, $h^3$, $h^4$) und Breite ($w^2$, $w^3$) groß genug sind, um den Meniskus am Einströmen in die zweite Zone (24d, e) zu hindern.

12. Reaktionsgefäß (20d) nach Anspruch 11, dadurch gekennzeichnet, daß der Kanal (100) eine Tiefe ($h^2$) besitzt, die mindestens um 0,02 mm größer ist als der Abstand ($h_d$) zwischen den beiden einander gegenüberliegenden Flüssigkeitstransportflächen (26d, 28d) in der ersten Zone (22d).

13. Reaktionsgefäß (20e) nach Anspruch 11, dadurch gekennzeichnet, daß die Meniskussteuermittel zwei einander gegenüberliegende Kanäle (100', 100") aufweisen, deren Tiefen ($h^3 + h^4$) zusammen mindestens um 0,015 mm größer sind als der Abstand ($h_e$) zwischen den beiden einander gegenüberliegenden Flüssigkeitstransportflächen (26e, 28e) in der ersten Zone (22e).

14. Reaktionsgefäß (20f) nach Anspruch 1, dadurch gekennzeichnet, daß das Reagens in der ersten Zone (22f) in einem immobilisierten Zustand vorliegt und die Fähigkeit besitzt, ein Material der Flüssigkeit sowie ein gekennzeichnetes Material zu binden, wobei die zweite Zone (24f) Flüssigkeit sowie darin enthaltenes Material aufnimmt, das nicht an das Reagens aus der ersten Zone (22f) gebunden ist.

15. Reaktionsgefäß (20f) nach Anspruch 14, dadurch gekennzeichnet, daß das Material ein in einer biologischen Flüssigkeit vorliegendes Immunogen und das Reagens die diesem Immunogen komplementäre Verbindung ist und daß in der zweiten Zone (24f) gekennzeichnetes Material nachgewiesen werden kann.

16. Reaktionsgefäß (20f) nach Anspruch 16, dadurch gekennzeichnet, daß in beiden Zonen (22f, 24f) gekennzeichnetes Material festgestellt werden kann.

17. Reaktionsgefäß (20g) nach Anspruch 1, dadurch gekennzeichnet, daß die erste Zone (22g) ein zellagglutinierendes Reagens enthält, das fest an mindestens einer (i) der einander gegenüberliegenden Flussigkeitstransportflächen und an (ii) Abstandselementen (30g) haftet, wobei die Einlaßmittel (46g) das Einführen von Gesamtblut in die erste Zone (22g) erlauben und die zweite Zone (24g) Plasma aus der ersten Zone (22g) aufnehmen kann und ein Nachweiselement (120g) mit mindestens einem für die Bestimmung eines Analyten des Plasmas spezifischen Nachweisreagens enthält.

18. Reaktionsgefäß (20h) nach Anspruch 1, dadurch gekennzeichnet, daß hinter der zweiten Zone (160) eine dritte Zone (24h) angeordnet ist und zweite Meniskussteuermittel (163) in einer Engstelle (162) zwischen der dritten Zone (24h) und der zweiten Zone (160) vorgesehen sind und daß die erste Zone (22h) ein zellagglutinierendes Reagens enthält, das an mindestens einer (i) der einander gegenüberliegenden Transportflächen und an (ii) Abstandselementen (30h) haftet, wobei die Einlaßmittel (46h) die Einführung von Gesamtblut in die erste Zone (22h) erlauben, die zweite Zone (160) Plasma aus der ersten Zone (22h) aufnehmen kann und die dritte Zone (24h) ein Nachweiselement (120h) mit mindestens einem für die Bestimmung eines Analyten des Plasmas spezifischen Nachweisreagens enthält.

19. Reaktionsgefäß (20h) nach Anspruch 18, dadurch gekennzeichnet, daß das Nachweiselement (120h) soviel Nachweisreagens enthält, wie nötig ist, um eine Farbveränderung zu bewirken, die der im Gesamtblut vorhandenen Menge an Plasma-Ammoniak und Plasma-Kreatinin proportional ist.

20. Verfahrung zur Vereinigung eines Reagens und einer ein Material enthaltenden Flüssigkeit in einem Reaktionsgefäß nach einem der Ansprüche 1-19, wobei eine Flüssigkeitsmenge in die erste Zone eingeführt und die Flüssigkeit mittels Kapillarwirkung durch die erste Zone transportiert wird, dadurch gekennzeichnet, daß die Flüssigkeit so angehalten wird, daß der die vordere Kante der Kapillarströmung bildende Meniskus an die Engstelle angrenzt, während eine Interaktion zwischen dem Reagens in der ersten Zone und dem Material erfolgt, daß in der ersten Zone auf die Flüssigkeit von außen ein so starker Druck ausgeübt wird, daß der Flüssigkeitsmeniskus aus der ersten in die zweite Zone gedruckt wird, und daß die Flüssigkeit unter Kapillarwirkung durch die zweite Zone transportiert wird.

21. Verfahren nach Anspruch 20, dadurch gekennzeichnet, daß das Reagens mit dem Material eine Verbindung eingehen kann und in der ersten Zone immobilisiert ist, daß die in die erste Zone eingeführte Flüssigkeitsmenge (i) eine Flüssigkeit mit einer unbekannten Menge eines nicht gekennzeichneten Materials und (ii) eine Flüssigkeit mit einer bekannten Menge eines gekennzeichneten Materials enthält, daß die Flüssigkeitsmenge in der ersten Zone angehalten wird, während das Reagens mit dem gekennzeichneten und dem nicht gekennzeichneten Material eine Verbindung eingeht, daß der Druck ausreicht, um ein Einströmen der Flüssigkeit und des in ihr enthaltenen, nicht an das Reagens gebundenen Materials in die zweite Zone zu bewirken und daß außerdem das freie gekennzeichnete Material in der zweiten Zone nachgewiesen wird, nachdem die Flüssigkeit in die zweite Zone geströmt ist.

22. Verfahren nach Anspruch 21, dadurch gekennzeichnet, daß anstelle des letztgenannten Arbeitsschritts das gebundene, immobilisierte gekennzeichnete Material in der ersten Zone nachgewiesen wird.

23. Verfahren nach Anspruch 21, dadurch gekennzeichnet, daß das Material ein in einer biologischen Flüssigkeit zu findendes Immunogen und das Reagens die diesem komplementäre Substanz ist. :

24. Verfahren nach Anspruch 21, 22 oder 23, dadurch gekennzeichnet, daß in weiteren Verfahrensschritten die nachgewiesene Menge an gekennzeichnetem Material mit der bekannten Menge verglichen und die Menge an unbekanntem nicht gekennzeichnetem Material berechnet wird, die zusammen mit dem gekennzeichneten Material an das Reagens gebunden wird.

25. Verfahren nach Anspruch 20, dadurch gekennzeichnet, daß die Flüssigkeit aus Gesamtblut besteht und das Material ein Analyt von Gesamtblut ist, daß das Reagens ein zellagglutinierendes Reagens ist, das in der ersten Zone immobilisiert ist, daß die zweite Zone mindestens ein für den Analyten spezifisches Nachweisreagens enthält, daß das Gesamtblut solange in der ersten Zone gehalten wird, bis die Blutzellen an das zellagglutinierende Reagens gebunden und damit von dem Blutplasma getrennt worden sind, daß der Druck so groß ist, daß er ein Einströmen des Plasmas, nicht aber der Zellen in die zweite Zone bewirkt, und daß als weiterer Verfahrensschritt in der zweiten Zone eine radiometrische Veränderung als Folge der Interaktion des Nachweisreagens mit dem Analyten festgestellt wird.

26. Verfahren nach Anspruch 25, dadurch gekennzeichnet, daß als weiterer Verfahrensschritt Plasma in eine zwischen der ersten und der zweiten Zone befindliche dritte Zone des Reaktionsgefäßes gedrückt wird, bevor es in die zweite Zone transportiert wird, wobei die dritte Zone ein Gas freisetzendes Reagens enthält, so daß Gas aus dem Plasma freigesetzt wird, ehe das Plasma in die zweite Zone gelangt.

27. Verfahren nach Anspruch 20, dadurch gekennzeichnet, daß das Reagens ein Ammoniakgas freisetzendes Reagens ist und sich in der ersten Zone befindet, daß die zweite Zone (i) ein Nachweisreagens, das Kreatinin mindestens zu Ammoniakgas zersetzt, und (ii) einen Indikator enthält, der bei Vorhandensein dieses Gases eine radiometrisch nachweisbare Veränderung bewirkt, daß die Flüssigkeit aus Ammoniak und Kreatinin enthaltendem Plasma besteht, daß das Plasma in der ersten Zone gehalten wird, während das Ammoniakgas von dem Gas freisetzenden Reagens freigesetzt wird und in die zweite Zone strömen kann, daß der Druck so hoch ist, daß er ein Einströmen des Plasmas in die zweite Zone bewirkt, daß als weiterer Verfahrensschritt das Gas in der zweiten Zone mittels des Indikators nachgewiesen wird, bevor Druck angewendet wird, und daß während des Transports des Plasmas durch die zweite Zone (i) das Plasma mit dem Nachweisreagens zur Reaktion gebracht wird, so daß weiteres Ammoniakgas entsteht, und (ii) dieses Gas mit dem Indikator zur Reaktion gebracht wird, wodurch die Kreatininkonzentration feststellbar ist.

28. Verfahren nach Anspruch 27, dadurch gekennzeichnet, daß als weitere Verfahrensschritte, die vor den obengenannten Verfahrensschritten erfolgen, eine Menge an Gesamtblut in die neben der ersten Zone befindliche und mit dieser kommunizierende dritte Zone des Reaktionsgefäßes gebracht wird, die Menge Gesamtblut solange in dieser dritten Zone gehalten wird, bis die Blutzellen an ein in der dritten Zone immobilisiertes, zellagglutinierendes Reagens gebunden und damit von dem Blutplasma getrennt worden sind, und auf die dritte Zone von außen soviel Druck ausgeübt wird, daß dadurch das Plasma in die erste Zone eingeführt wird.

## Revendications

1. Enceinte pour réactions (20-20h) comprenant des éléments (42-42h, 44-44h, 30, 30a, b, d-h) qui constituent une première zone (22-22h) et une seconde zone (24, 24c-g, 160) pour recevoir un liquide, des moyens d'introduction, (46, 46a, b, d-h) pour laisser passer du liquide dans cette première zone (22-22h), et un chenal (60, 60c-h) qui relie ces zones (22-22h, 24, 24c-g, 160) et qui présente une section droite de taille capillaire, ces éléments, (42-42h, 44-44h, 30, 30a, b, d-h) présentant des surfaces de transfert de liquide opposées (26-26h, 28-28h) et des surfaces de parois latérales (32, 32c, d, f-h, 34, 34f, h) qui coopèrent avec ces surfaces de transfert de liquide opposées (26, 26h, 28-28h) pour délimiter ces zones (22-22h, 24, 24c-g, 160) et ce chenal (60, 60c-h), ces surfaces de transport de liquide opposées, (26-26h, 28-28h) étant séparées par une distance qui est apte à induire un écoulement capillaire de ce liquide dans au moins une partie de cette première zone (22-22h) contiguë à ce chenal (60, 60c-h), et ce chenal (60, 60c-h,) étant muni de moyens de maîtrise du ménisque (62, 62c, f-h, 100, 100′, 100″), ayant des bords opposés, nets (62, 62f-h) caractérisé en ce que la première zone au moins (22-22h) contient un réactif capable d'interagir avec un produit contenu dans un liquide, en ce que les moyens de maîtrise du ménisque comprennent en plus lesdites surfaces de parois latérales (32, 32c, d, f-h, 34, 34f, h) ou lesdites surfaces de transfert de liquide opposées (26c, 28c) à l'intérieur du chenal et qui convergent dans la direction d'écoulement du fluide et en ce que lesdits moyens de contrôle du ménisque servent à réguler le ménisque constituant le bord d'attaque de l'écoulement capillaire de manière à arrêter le liquide qui progresse par capillarité de cette première zone (22-22h) dans cette seconde zone (24, 24c-g, 160) et à ne permettre l'écoulement du liquide dans cette seconde zone (24, 24c-g, 160) seulement lorsqu'une pression suffisante, engendrée de l'extérieur, est communiquée au liquide de la première zone (22-22h).

2. Enceinte pour réactions (20-20h) conforme à la revendication 1, caractérisée en ce que l'un de ces éléments (42-42h) est notablement rigide.

3. Enceinte pour réactions (20-20h) conforme à la revendication 1 ou 2, caractérisée en ce que cette distance est apte à induire un écoulement capillaire de ce liquide de ces moyens d'introduction (46, 46a, b, d-h) vers ce chenal (60, 60c-h).

4. Enceinte pour réactions (20-20h) conforme à l'une quelconque des revendications 1-3, caractérisée en ce que l'une au moins de ces surfaces opposées (26, 26h, 28-28h) comprend, dans l'une ou ces deux zones (22-22h, 24, 24c-g, 160), un produit relativement non mouillable revêtu d'un agent tensioactif soluble dans le liquide et propre à rendre ces surfaces mouillables par le liquide.

5. Enceinte pour réactions (20-20b, 20f-h) conforme à la revendication 1, caractérisée en ce que ces surfaces de parois latérales (32, 32f-h, 34, 34f, h) dans cette seconde zone (24, 24f-g, 160) voisines de ce chenal (60, 60f-h) divergent de ces bords (62, 62f-h) dans cette seconde zone (24, 24f-g, 160) avec une valeur constante.

6. Enceinte pour réactions (20-20b, 20f-h) conforme à la revendication 1, caractérisée en ce que ces surfaces de parois latérales (32, 32f, 34, 34f) de cette seconde zone (24, 24f) divergent de ces bords (62, 62f) avec une valeur qui croît avec l'éloignement à ces bords (62, 62f).

7. Enceinte pour réactions (20c) conforme à l'une quelconque des revendications 1-4, caractérisée en ce que ces moyens de maîtrise du ménisque comprennent un couple de bords opposés (62c) dans ces surfaces opposées (26c, 28c) qui provoquent une diminution dans cette distance de séparation (h'').

8. Enceinte pour réactions (20c) conforme à la revendication 7, caractérisée en ce que cette diminution de distance entre ces deux surfaces (26c, 28c), a une valeur qui croît à mesure que ces moyens de maîtrise du ménisque (62c) approchent de ces moyens d'introduction.

9. Enceinte pour réactions (20-20c) conforme à l'une quelconque des revendications 1-8, caractérisée en ce que ces moyens de maîtrise du ménisque comprennent des bords opposés (62, 62c) dont la longueur dans le sens de l'écoulement du liquide qui les traverse n'est pas supérieure à 0,3 mm.

10. Enceinte pour réactions (20, 20a, b, d, e) conforme à l'une quelconque des revendications 1-4, caractérisée en ce que ces éléments (42, 42a, b, d, e, 44, 44a b, d, e, 30, 30a, b) qui constituent ces zones (22, 22a, b, d, e, 24, 24d, e) comprennent un élément de support (44, 44a, b, d, e) et un élément de couverture (42, 42a, b, d, e) qui sont séparés d'une première distance (h', $h_d'$, $h_e'$) dans cette seconde zone (24, 24d, e) et d'une seconde distance (h, $h_d$, $h_e$) dans cette première zone (22, 22a, b, d, e), cette première distance (h', $h_d'$, $h_e'$) étant inférieur à cette seconde distance (h, $h_d$, $h_e$) d'une quantité propre à attirer préférentiellement ce liquide, en réponse à cette application d'une pression au liquide, dans cette seconde zone (24, 24d, e) à partir de cette première zone (22, 22a, b, d, e) et à confiner le liquide ainsi attiré dans cette seconde zone (24, 24d, e).

11. Enceinte pour réactions (20d, e) conforme à l'une quelconque des revendications 1-4, caractérisée en ce que ces moyens de maîtrise du ménisque comprennent un canal (100, 100', 100'') dans l'une au moins de ces surfaces opposées (26d-e, 28d-e) qui présente une profondeur ($h^2$, $h^3$, $h^4$) et une largeur ($w^2$, $w^3$) suffisante pour arrêter le ménisque de s'écouler dans cette seconde zone (24d, e).

12. Enceinte pour réactions (20d) conforme à la revendication 11, caractérisée en ce que ce canal (100) a une profondeur ($h^2$) qui est d'au moins 0,02 mm plus grande que la distance ($h_d$) entre ces deux surfaces opposées (26d, 28d) dans cette première zone (22d).

13. Enceinte pour réactions (20e) conforme à la revendication 11, caractérisée en ce que ces moyens de maîtrise du ménisque comprennent deux canaux opposés (100', 100'') qui ont une profondeur combinée ($h^3 + h^4$) qui est au moins supérieure de 0,015 mm à la distance ($h_e$) entre ces surfaces opposées (26e, 28e) dans cette première zone (22e).

14. Enceinte pour réactions (20f) conforme à la revendication 1, caractérisée en ce que ce réactif est dans un état immobilisé dans cette première zone (22f) et possède l'aptitude de se lier à un produit du liquide et à un produit catalogué, cette seconde zone (24f) étant adaptée à recevoir du liquide et du produit qu'il contient qui ne sont pas liés à ce réactif de la première zone (22f).

15. Enceinte pour réactions (20f) conforme à la revendication 14, caractérisée en ce que ce produit est un immunogène présent dans un liquide biologique, ce réactif est un immunogène complémentaire, et en ce que cette seconde zone (24f) est conçue pour la détection du produit catalogué.

16. Enceinte pour réactions (20f) conforme à la revendication 15, caractérisée en ce que ces deux zones (22f, 24f) sont conçues pour la détection de produit catalogué.

17. Enceinte pour réactions (20g) conforme à la revendication 1, caractérisée en ce que cette première zone (22g) contient un réactif agglutinant des globules adhérant immobilement à au moins un de i) ces surfaces opposées et ii) éléments d'entretoise (30g) ces moyens d'introduction (46g) permettant de faire passer du sang complet dans cette première zone (22g) et cette seconde zone (24g) étant conçue pour recevoir du plasma de cette première zone (22g) et contenant un élément de détection (120g) comprenant au moins un réactif de détection spécifique de l'analyse d'un analyte du plasma.

18. Enceinte pour réactions (20h) conforme à la revendication 1, caractérisée en ce qu'une troisième zone (24h) est disposée en aval de cette seconde zone (160) et des deuxièmes moyens de maîtrise du ménisque (163) sont logés dans un canal (162) disposé entre cette troisième zone (24h) et cette seconde zone (160), et en ce que cette première zone (22h) contient un réactif agglutinant des globules adhérant immobilement à au moins un de i) ces surfaces opposées et ii) éléments d'entretoise (30h), ces moyens d'introduction (46h) permettant de faire passer du sang complet dans cette première zone (22h), cette seconde zone (160) étant conçue pour recevoir du plasma de cette première zone (22h), et cette troisième zone (24h) contenant un élément de détection (120h) comprenant au moins un réactif de détection spécifique de l'analyse d'un analyte du plasma.

19. Enceinte pour réactions (20h) conforme à la revendication 18, caractérisée en ce que cet élément de détection (120h) comprend des réactifs de détection suffisants pour engendrer un changement de coloration proportionnelle à la quantité d'ammoniaque du plasma ainsi que la quantité de créatinine du plasma qui sont présentes dans le sang complet.

20. Procédé pour associer ensemble dans une enceinte de réactions selon l'une quelconque des revendications 1 à 19 un réactif et un liquide contenant un produit, ce procédé comprenant les étapes suivantes :

le dépôt d'une quantité de ce liquide dans cette première zone, et

le transfert de ce liquide au travers de cette première zone par capillarité,

caractérisé en ce que l'on retient ce liquide avec le ménisque formant le bord d'attaque de l'écoulement capillaire contigu à ce chenal alors que le réactif interréagit avec ce produit,

l'application à ce liquide dans la première zone d'une pression suffisante, engendrée de l'extérieur, pour pousser le ménisque du liquide de la première zone dans la seconde zone et

le transfert de ce liquide au travers de la seconde zone par capillarite.

21. Procédé conforme à la revendication 20, caractérisé en ce que ce réactif est capable de se lier à ce produit et est immobilisé dans cette première zone, cette quantité de liquide introduit dans cette première zone contient i) un liquide contenant une quantité inconnue d'un produit non catalogué et ii) un liquide contenant une quantité connue d'un produit catalogué, cette quantité de liquide est retenue dans cette première zone alors que le réactif se lie au produit catalogué et au produit non catalogué, cette pression est suffisante pour forcer le liquide et le produit qu'il contient qui est dépourvu de ce réactif à commencer à s'écouler à cette seconde zone et comprenant en outre l'étape consistant en la détection du produit catalogué exempt dans cette seconde zone après que le liquide s'est déplacé dans la seconde zone.

22. Procédé conforme à la revendication 21, caractérisé en ce qu'à la place de la dernière étape précitée on procède à la détection du produit catalogué immobilisé lié dans cette première zone.

23. Procédé conforme à la revendication 21, caractérisé en ce que ce produit est un immunogène rencontré dans un liquide biologique, et ce réactif est le complément de cet immunogène.

24. Procédé conforme à la revendication 21, 22 ou 23 suivi des étapes consistant en

la comparaison de la quantité de produit catalogue détectée par rapport à cette quantité connue et

en le calcul de la quantité inconnue de produit non catalogué qui est en concurrence avec le produit catalogué pour ce réactif.

25. Procédé conforme à la revendication 20, caractérisé en ce que ce liquide est du sang complet et ce produit est un analyte du sang complet, ce réactif est un réactif agglutinant des globules qui est immobilisé dans cette première zone, cette seconde zone contient au moins un réactif de détection spécifique de cet analyte, ce sang complet est retenu dans cette première zone pendant une durée suffisante pour que les globules sanguins s'associent à ce réactif agglutinant des globules et ainsi se séparent du plasma sanguin, cette pression est suffisante pour forcer le plasma, mais non les globules, à s'écouler dans cette seconde zone, et en outre comprenant l'étape consistant en la détection dans cette seconde zone d'un changement radiométrique résultant de l'interaction entre le réactif de détection et cet analyte.

26. Procédé conforme à la revendication 25, caractérisé par l'étape supplémentaire consistant en le forcement de ce plasma dans une troisième zone de l'enceinte entre cette première et cette seconde zones avant qu'il soit forcé dans cette seconde zone, la troisième zone contenant un réactif libérateur de gaz de manière que le gaz soit libéré du plasma avant que le plasma soit forcé dans la seconde zone.

27. Procédé conforme à la revendication 20, caractérisé en ce que ce réactif est un réactif libérant du gaz ammoniac et est dans cette première zone, cette seconde zone comprend i) un réactif de détection qui décompose la créatinine en au moins du gaz ammoniac et ii) un indicateur qui engendre un changement détectable radiométriquement du fait de ce gaz, ce liquide est du plasma contenant de l'ammoniaque et de la créatinine, ce plasma est retenu dans cette première zone alors que le gaz ammoniac est libéré par ce réactif libérant un gaz et autorisé à s'écouler dans cette seconde zone, cette pression est suffisante pour forcer le plasma à commencer à s'écouler dans cette seconde zone, et en outre comprenant les étapes consistant en

la détection du gaz dans cette seconde zone avec cet indicateur avant d'appliquer cette pression, et

alors que ce plasma est en cours de transfert au travers de cette seconde zone, la réaction i) du plasma avec ce réactif de détection pour former du gaz ammoniac supplémentaire, et ii) du gaz supplémentaire avec cet indicateur de manière à détecter la concentration de créatinine.

28. Procédé conforme à la revendication 27, caractérisé par les étapes supplémentaires, conduites avant les étapes énumérées précédemment, consistant en

le dépôt d'une quantité de sang complet dans une troisième zone de l'enceinte disposée au voisinage de et en communication fluide avec cette première zone ;

la retenue de cette quantité de sang complet dans cette troisième zone pendant une durée suffisante pour que les globules sanguins s'associent au réactif agglutinant des globules qui est immobilisé dans cette troisième zone et ainsi se séparent du plasma sanguin ;

l'application dans cette troisième zone d'une pression engendrée de l'extérieur en grandeur suffisante pour faire passer ce plasma dans cette première zone.

FIG. 1

FIG. 2

FIG. 3

FIG. 4

FIG. 5

FIG.6

FIG.7

FIG.8

FIG.9

FIG.10

FIG.11

FIG.12 A

FIG.12 B

FIG.13

FIG.14

FIG.15